# EUROPEAN PATENT APPLICATION

(11) **EP 2 886 104 A1**
(43) Date of publication of application: **24.06.2015**
(21) Application number: 14197222.4
(22) Date of filing: 10.12.2014
(51) Int. Cl.: A61K 9/00, A61K 31/728, A61K 31/737, A61K 31/7008

(54) **An intra-articular gel**

(30) Priority: 11.12.2013 TR 201314567; 17.11.2014 TR 201413520
(71) Applicant: Patir, Suleyman, Etimesgut, Ankara (TR); Dikmen, Alican, Ankara (TR); Burak, Burhanettin Can, Istanbul (TR); Konukoglu, Osman Burak, Istanbul (TR)
(72) Inventor: Patir, Suleyman, Etimesgut, Ankara (TR); Dikmen, Alican, Ankara (TR); Burak, Burhanettin Can, Istanbul (TR); Konukoglu, Osman Burak, Istanbul (TR)
(74) Representative: Cicogna, Franco

(57) **Abstract**

The present invention relates to an intra-articular gel which has a preventive and therapeutic characteristic in intra-articular cartilage destruction and degenerative meniscopathy in early osteoarthritis; comprises sodium hyaluronate, chondroitin sulfate and N-Acetyl glucosamine; and which is a biomaterial implant.

## Description

### Technical Field

The present invention relates to an intra-articular gel which has a preventive and therapeutic characteristic in intra-articular cartilage destruction and degenerative meniscopathy in early osteoarthritis; comprises sodium hyaluronate, chondroitin sulfate and N-Acetyl glucosamine and which is a bio-material implant.

### Background of the Invention

Proteoglycanes are one of the basic structures composing cartilage matrix. Enzymatic degeneration in proteoglycanes is one of the bio-chemical changes in osteoarthritis. And this causes proteoglycanes to be synthesized by articular chondrocytes insufficiently and disintegrated by metalloproteinase enzymes in the matrix. Proteoglycanes consist of interconnection of core protein (aggrecan) monomers and then bonding of these proteins, chondroitin and keratan sulfate molecules to glucosaminoglycanes by covalent bonds. Side chains provide resistance of cartilage matrix to hydrolysis and compression.

Among non-surgical treatment options of early osteoarthritis and cartilage degeneration, intra-articular preparations are used most commonly. These are preparations which comprise hyaluronic acid, mixture of hyaluronic acid-chondroitin sulfate and are used today most commonly. Preventive and therapeutic characteristics of these preparations have been detected in early osteoarthritis, cartilage destruction cases and degenerative meniscopathy in the patent documents to be described hereinafter, academic publications and clinical trials.

Today, products which are used for treatment of osteoarthritis, cartilage degeneration in joints comprise sodium hyaluronate and sodium hyaluronate-chondroitin sulfate combination. The patent documents no. US3697625A, US4216204A, US4782,046A, US4801619A, US4808576A, US4837024A, US5141928A, US5364845A, US5442053A, US5466823A, US5587363, US5756529A, US5840715A, US5916565A, US5929050A, US6127356A, US6271213B1, US6432929B1, US6492349B1, US6583123B2, US6632804B2, US6645948B2, US6906044B2, US6949525B2, US7845629B2, US7803787B2 and GB2317109A of the prior art disclose applications used in treatment of osteoarthritis and treatment of cartilage degeneration in joints.

In addition, the United States patent document no. US20060110459 discloses a triple natural polymer viscoelastic composition. In the said composition, glucosamine sulfate, sodium hyaluronate and chondroitin sulfate are combined and the composition is used as viscoelastic agent at surgical sites. The said composition is used as a protective agent to ocular tissues and also enables to alleviate pain and inflammation. The said composition which can be also used as an intraocular and/or intra-articular agent comprises monosodium phosphate, disodium phosphate, sodium chloride, glucosamine sulfate, sodium hyaluronate and chondroitin sulfate in sample usage.

Besides, the United States patent document no. US2008139500 also discloses a composition which is used in treatment of osteoarthritis in knee and hip joints. The said composition enables to make transaction by an intra-articular operation without needing a knee or hip joint replacement surgery. The composition includes monomer or monomers, sodium hyaluronate, glucosamide sulfate, chondroiten sulfate and chondroiten sulfate.

In addition, the European Patent document no EP1560588 B1 discloses a composition of N-Acetyl glucosamine and sodium hyaluronate.

The publication called "Stimulation of proteoglycan Production by Glucosamine Sulfate in chondrocytes Isolated from Human Osteoarthritic Articual Cartilage in Vitro" and published by Bassleer et al. in 1998; the publication called "Osteoarthritis and Cartilage"; the publication called "Hexosamine Metabolism" and published by Capps et al.; the publication called "Characterization of the Effect of High Molecular Weight Hyaluronan on Trans-Synovial Flow in Rabbit Knee" and published by Coleman et al. in 1999; the publication called "Efficacy of intraarticular hyaluronic acid injections in knee" and published by Evanich et al.; the publication called "Viscosupplementation of osteoarthritic knees with hylan: A treatment study schedule" and published by Scale et al.; the publication called "Viscosupplementation with hylan for the treatment of osteoarthritis: Findings from clinical practice in Canada" and published by Lussier et al.; the publication called "Hyaluronic acid in the treatment of osteoarthritis of the knee, Rheumatology" and published by Huskisson et al.; the publication called "Chondroprotective activity of N-acetylglucosamine in rabbits with experimental Osteoarthritis" and published by Shikhman AR, Amiel D, D'Lima D, Hwang SB, Hu C, Xu A, Hashimoto S, Kobayashi K,Sasho T, Lotz MK. are documents which are in the state of the art and disclose application, apparatus, etc. techniques used in treatment of osteoarthritis.

### Summary of the Invention

An objective of the present invention is to realize an intra-articular gel which enables fast and effective treatment, alleviating pain and structuring and protection of cartilage by addition of N-Acetyl glucosamine.

### Detailed Description of the Invention

The inventive intra-articular gel used for osteoarthritis, cartilage degeneration in joints comprises sodium hyaluronate, chondroitin sulphate, N-Acetyl glucosamine, NaH₂PO₄ (monosodiumphosphate), Na₂HPO₄ (disodiumphosphate), NaCl (sodium chloride) and injection water.

There is 0.042% NaH₂PO₄, 0.18% Na₂HPO₄, 0.40% NaCl, 2.84% chondroitin sulphate, 1.32% sodium hyaluronate, 0.47% N-Acetyl glucosamine and 94.71% injection water on a dose basis in the intra-articular gel.

In another preferred embodiment there is 2mg NaH₂PO₄, 0,45mg Na₂HPO₄, 4,3mg NaCl, 30mg chondroitin sulphate, 16mg sodium hyaluronate, 30mg N-Acetyl glucosamine and injection water on a 1 mL basis in the intra-articular gel.

In a preferred embodiment, molecular weight of chondroitin sulfate in the intra-articular gel is between 2000-10000 Daltons. In a preferred embodiment, molecular weight of sodium hyaluronate in the intra-articular gel is 1.8 million Daltons. Besides, there is 15 - 20 mg N-Acetyl glucosamine in a dose.

In another preferred embodiment molecular weight of chondroitin sulfate in the intra-articular gel is between 10000-30000 Daltons and there is 10mg-40mg chondroitin sulfate on 1 mL. In a preferred embodiment molecular weight of sodium hyaluronate in the intra-articular gel is between 1.8 million - 3.0 million Daltons. There is hyaluronic acid between the range of 12mg-21 mg on 1 mL. In addition there is 5mg-40mg N-Acetyl glucosamine on 1 mL.

In example formulations given for the inventive intra-articular gel, amounts of a gel comprising 1 ml are given.

**Formulation 1**

| | |
|---|---|
| NaH₂PO₄ | 0.45 mg |
| Na₂HPO₄ | 2 mg |
| NaCl | 4.3 mg |
| Chondroitin Sulfate | 30 mg |
| Sodium Hyaluronate | 16 mg |
| N-Acetyl Glucosamine | 5 mg |

**Formulation 2**

| | |
|---|---|
| NaH₂PO₄ | 0.45 mg |
| Na₂HPO₄ | 2 mg |
| NaCl | 4.3 mg |
| Chondroitin Sulfate | 30 mg |
| Sodium Hyaluronate | 16 mg |
| N-Acetyl Glucosamine | 30 mg |

The present invention is an intra-articular gel which has a preventive and therapeutic characteristic in intra-articular cartilage destruction and degenerative meniscopathy in early osteoarthritis; comprises derivatives of sodium hyaluronate, chondroitin sulfate and glucosamine; and which is a bio-material implant. Hyaluronic acid is a polymer consisting of N-Acetyl glucosamine and glucronic acid units. In vitro studies indicated that glucosamine supports production of cartilage matrix components. In addition, glucosamine triggers synthesis of hyaluronic acid. Researches indicated that glucosamine prevents destruction of chondrocyte and collagen by inhibiting lipooxidation mechanism.

It is possible to develop a great variety of embodiments of the inventive intra-articular gel within these basic concepts, it cannot be limited to examples disclosed herein and it is essentially according to the claims.

## Claims

1. An intra-articular gel used in osteoarthritis, cartilage degeneration in joints, **comprising** sodium hyaluronate, chondroitin sulphate, N-Acetyl glucosamine, NaH₂PO₄ (monosodiumphosphate), Na₂HPO₄ (disodiumphosphate), NaCl (sodium chloride), injection water;
**characterized in that**
- there is sodium hyaluronate between 12mg and 21 mg on 1 mL basis in it.

2. An intra-articular gel according to claim 1, **characterized in that** molecular weight of Chondroitin Sulfate in it is between 10000-30000 Daltons and there is 10mg-40mg Chondroitin Sulfate on 1 mL gel.

3. An intra-articular gel according to any of the preceding claims, **characterized in that** molecular weight of Sodium Hyaluronate is between 1.8 million-3.0 million Daltons and there is Sodium Hyaluronate between the range of 12mg and 21 mg on 1 mL gel.

4. An intra-articular gel according to any of the preceding claims, **characterized in that** there is 5mg-40 mg N-Acetylglucosamine in 1 mL gel.
